# EUROPÄISCHE PATENTSCHRIFT

(11) **EP 2 403 359 B1**
(45) Veröffentlichungstag und Bekanntmachung des Hinweises auf die Patenterteilung: **23.01.2013**
(21) Anmeldenummer: 10706262.2
(22) Anmeldetag: 04.03.2010
(51) Int. Cl.: A23L 1/275, A23K 1/16, C07C 403/24

(54) **PULVERFÖRMIGE ZUSAMMENSETZUNG VON ASTAXANTHIN-DERIVATEN I**
POWDERY COMPOSITION OF ASTAXANTHIN DERIVATIVES I
COMPOSITION PULVÉRULENTE DE DÉRIVÉS D'ASTAXANTHINE I

(30) Priorität: 05.03.2009 EP 09154438
(43) Veröffentlichungstag der Anmeldung: 11.01.2012
(73) Patentinhaber: BASF SE, 67056 Ludwigshafen (DE)
(72) Erfinder: FELDTHUSEN JENSEN, Jesper, 55268 Nieder-Olm (DE); KÖPSEL, Christian, 69469 Weinheim (DE); END, Lutz, 68526 Ladenburg (DE); BRANDS, Mario, 67063 Ludwigshafen (DE); ENGEL, Robert, 67346 Speyer (DE); GOTTSCHALK, Thomas, 68165 Mannheim (DE); PELLETIER, Wolf, 76879 Ottersheim (DE)
(86) Internationale Anmeldenummer: PCT/EP2010/052758
(87) Internationale Veröffentlichungsnummer: WO 2010/100228

(56) Entgegenhaltungen:
- EP-A1- 1 227 082
- WO-A1-03/066583
- WO-A1-2006/125591

## Beschreibung

Die vorliegende Erfindung betrifft feste pulverförmige Zusammensetzungen von Astaxanthin-Derivaten sowie die Verwendung der Zusammensetzungen zur Herstellung von Lösungen des Astaxanthin-Derivats in für Nahrungsmittel geeigneten Ölen sowie zur Herstellung von Futtermitteln. Die Erfindung betrifft auch ein Verfahren zur Herstellung derartiger Zusammensetzungen und ein Verfahren zur Herstellung von Futtermitteln unter Verwendung der pulverförmigen Zusammensetzungen.

Astaxanthin (3,3'-Dihydroxy-ß,ß-carotin-4,4'-dion) und seine Derivate, insbesondere seine Ester, sind als Nahrungs- und Futtermittelzusatzstoffe von Interesse. So wird Astaxanthin in großem Umfang bei der Aufzucht von Speisefischen in Aquakulturen (Fischfarmen) als Futtermittelzusatz eingesetzt. Aufgrund der vitaminartigen Eigenschaft von Astaxanthin und seinen Derivaten wirken sie sich vorteilhaft auf die Gesundheit der Fische in den Aquakulturen aus und fördern deren Fruchtbarkeit. Zudem bewirken Astaxanthin und seine Derivate eine Intensivierung der Färbung von Fleisch und Haut der Fische, was nicht zuletzt aus ästhetischen und kulinarischen Gründen wünschenswert ist.

Problematisch sind die geringe Wasserlöslichkeit von Astaxanthin und seinen Derivaten und ihre damit einhergehende schlechte Bioverfügbarkeit. Aus diesem Grund können Astaxanthin und seine Derivate nicht als solche eingesetzt werden, sondern müssen in eine Formulierung überführt werden, die eine hinreichende Bioverfügbarkeit dieser Substanzen gewährleistet. Aufgrund der chemischen Instabilität vom Astaxanthin und seinen Derivaten stellt jedoch die Formulierung dieser Verbindungen eine besondere Herausforderung dar.

Für verschiedene Anwendungszwecke, insbesondere zur Futtermittelherstellung, hat es sich grundsätzlich als vorteilhaft erwiesen, Astaxanthin oder seine Derivate in Form von verdünnten Lösungen in für Nahrungsmittel geeigneten, flüssigen Ölen bereitzustellen. Diese verdünnten Lösungen können dann bei der Futtermittelherstellung verwendet werden und gewährleisten eine hinreichende Bioverfügbarkeit in dem Futtermittel. Bei der Herstellung derartiger Lösungen erweist sich die schlechte Lösungskinetik des Astaxanthins als problematisch.

Die US 4,522,743 beschreibt ein Verfahren zur Herstellung pulverförmiger Carotinoid-Zusammensetzungen mit Partikelgrößen von < 0,5 µm, bei dem man das Carotinoid zunächst in einem flüchtigen, mit Wasser mischbaren Lösungsmittel bei erhöhter Temperatur löst, das Carotinoid aus der so erhaltenen Lösung durch rasches Eintragen in eine wässrige Lösung eines quellbaren Schutzkolloids fällt und die so erhaltene Suspension sprühtrocknet. Das pulverförmige Carotinoid ist in Wasser dispergierbar. Ölige Lösungen werden nicht beschrieben.

Die EP 1228705 und EP 1219292 beschreiben in Wasser dispergierbare Trockenpulver des Astaxanthins, die als Schutzkolloid ein Soja-Protein enthalten.

Die EP 845503 beschreibt flüssige mit Öl mischbare Carotinoid-Zubereitungen in Form einer Wasser-in-Öl-Emulsion, worin die dispergierte wässrige Phase schutzkolloidstabilisierte Carotinoid-Partikel in suspendierter Form enthält. Das Verfahren zur Herstellung dieser Zubereitungen ist vergleichsweise aufwändig. Zudem ist der Anteil an Carotinoid vergleichsweise gering.

Die WO 03/102116 beschreibt ölige Lösungen von Carotinoiden wie Astaxanthin. Ihre Herstellung erfolgt durch Lösen des Carotinoids in einem organischen Lösungsmittel wie N-Methylpyrrolidon in Anwesenheit eines lipophilen Dispergiermittels und Entfernen des Lösungsmittels. Das erhaltene Pulver wird dann in einem Öl, beispielsweise Fischöl, gelöst.

Die WO 03/066583 beschreibt neue Derivate des Astaxanthins und deren Verwendung bei der Herstellung von Futtermitteln.

Die WO 2006/125591 beschreibt die Herstellung von öligen Carotinoid-Lösungen, beispielsweise Lösungen des Astaxanthins, bei der man zunächst eine Suspension des Carotinoids in der Ölphase bereitstellt, die Suspension für eine kurze Zeit auf hohe Temperaturen, z. B. im Bereich von 100 bis 230°C erhitzt, um das Carotinoid in Lösung zu bringen, und die heiße Lösung mit einem kalten Öl vermischt. Zur Herstellung von öligen Lösungen des Astaxanthins werden regelmäßig Temperaturen oberhalb 160°C angewendet. Dieses Verfahren ist mit einer Reihe von Nachteilen verbunden. Zum einen führt das Erhitzen aufgrund der chemischen Labilität von ß-Carotinoiden unter anderem zu einer unerwünschten cis-trans-Isomerisierung der exocyclischen Doppelbindungen und damit zu einem Aktivitätsverlust. Zudem erweist sich die Bereitstellung öliger Suspensionen des Astaxanthins durch Vermahlen von Astaxanthin in Öl als problematisch.

Der vorliegenden Erfindung liegt die Aufgabe zu Grunde, geeignete Formulierungen von Astaxanthin oder einem Astaxanthin-Derivat bereitzustellen, die es erlauben, Astaxanthin oder das Astaxanthin-Derivat in ein für Nahrungsmittel geeignetes Öl einzuarbeiten. Die Formulierungen sollten einfach herzustellen sein und einen hohen Gehalt an Astaxanthin bzw. einen hohen Gehalt eines für Nahrungsmittel geeigneten Astaxanthin-Derivats aufweisen. Zudem sollte das darin enthaltene Astaxanthin einen hohen Gehalt an all-trans-Isomer aufweisen.

Die ältere internationale Patentanmeldung PCT/EP2008/061384 beschreibt ein Verfahren zur Herstellung von öligen Lösungen des Astaxanthins in für Nahrungsmittel geeigneten Ölen, bei denen man bestimmte Astaxanthin-Derivate der im Folgenden angegebenen Formel I zunächst in eine Suspension in einem essbaren Öl überführt, wobei die Partikel des Astaxanthin-Derivats in der Suspension einen volumenmittleren Teilchendurchmesser, bestimmt durch Fraunhofer-Beugung, im Bereich von 0,5 bis 50 µm aufweisen. Diese Suspensionen sind typischerweise bei Temperaturen von 25 °C flüssig und können in einfacher Weise mit flüssigen, für Nahrungsmittel geeigneten Ölen verdünnt werden, wobei sich die verwendeten Astaxanthin-Derivate aufgrund ihrer vorteilhaften Lösungskinetik und der geringen Partikelgröße rasch in dem zur Verdünnung eingesetzten Öl auflösen, ohne dass es der Anwendung von Temperaturen oberhalb 100 °C und/oder größerer Mengen an Emulgatoren bedarf.

Es wurde nun überraschenderweise gefunden, dass Astaxanthin-Derivate, die zu wenigstens 70 Gew.-% aus einer Verbindung der im Folgenden definierten Formel I bestehen, anders als andere Astaxanthin-Derivate, sich besonders einfach durch trockenes Vermahlen in Pulver überführen lassen. Außerdem wurde gefunden, dass solche Pulver, die ein solches Astaxanthin-Derivat in einer Menge von wenigstens 70 Gew.-% enthalten, eine vergleichbar gute Lösungskinetik in für Nahrungsmittel zugelassenen Ölen aufweisen, wenn wenigstens 90 Gew.-% der Pulverteilchen einen Teilchendurchmesser unterhalb 100 µm, insbesondere nicht mehr als 50 µm und speziell nicht mehr als 20 µm aufweisen und die Pulverteilchen eine mittlere Teilchengröße (Volumenmittel, D_{4,3}-Wert ) im Bereich von 0,5 bis 50 µm, insbesondere im Bereich von 0,5 bis 30 µm und speziell im Bereich von 0,5 bis 10 µm aufweisen.

Dementsprechend betrifft die vorliegende Erfindung pulverförmige Zusammensetzungen, die wenigstens ein Astaxanthin-Derivat in einer Menge von wenigstens 50 Gew.- %, z. B. 50 bis 100 Gew.-%, insbesondere 70 bis 100 Gew.-%, bezogen auf das Gesamtgewicht der Zusammensetzung enthalten, worin wenigstens 90 Gew.-% der Pulverteilchen einen Teilchendurchmesser unterhalb 100 µm, insbesondere nicht mehr als 50 µm und speziell nicht mehr als 20 µm aufweisen und die Pulverteilchen eine mittlere Teilchengröße (Volumenmittel, D_{4,3}-Wert) im Bereich von 0,5 bis 50 µm, insbesondere im Bereich von 0,5 bis 30 µm und speziell im Bereich von 0,5 bis 10 µm aufweisen und wobei das Astaxanthin-Derivat zu wenigstens 70 Gew.-%, insbesondere zu wenigstens 80 Gew.-%, besonders zu wenigstens 90 Gew.-% oder zu wenigstens 96 Gew.-% aus wenigstens einer Verbindung der Formel I 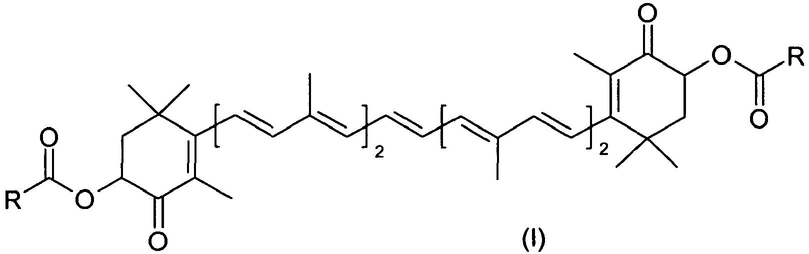 besteht, worin R für einen Rest der Formel 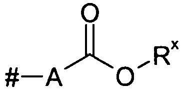 steht, worin # die Anknüpfung an die Carbonylgruppe bedeutet, A für -CH₂CH₂- oder für -CH=CH- steht und R^{x} für C₁-C₄ Alkyl steht.

Die Erfindung ist mit einer Reihe von Vorteilen verbunden. Zum einen lassen sich unter Verwendung der erfindungsgemäßen pulverförmigen Zusammensetzungen in einfacher Weise Lösungen von Astaxanthin-Derivaten in für Nahrungsmittel geeigneten Ölen durch Einarbeiten der erfindungsgemäßen Zusammensetzung in das für Nahrungsmittel geeignete Öl herstellen. Anders als in dem in WO 2006/125591 beschriebenen Verfahren bedarf es hierzu grundsätzlich nicht der Anwendung von Temperaturen oberhalb von 100°C. Vielmehr kann man unter Verwendung der erfindungsgemä-βen Zusammensetzungen verdünnte Lösungen des Astaxanthin-Derivats in dem für Nahrungsmittel geeigneten Öl bei Temperaturen unterhalb von 100°C, insbesondere bei maximal 90 °C oder gar bei maximal 80 °C, z. B. bei Temperaturen im Bereich von 20 bis 90 °C insbesondere von 30 bis 80 °C, herstellen. Aufgrund der niedrigen Temperaturen zeichnen sich die auf diese Weise erhältlichen Lösungen durch einen sehr hohen Anteil an all-trans-Isomeren aus, der in der Regel oberhalb von 80 %, insbesondere oberhalb von 90 %, bezogen auf das in der Formulierung enthaltene Astaxanthin-Derivat liegt. Ferner lassen sich unter Verwendung von Astaxanthin-Derivaten, die zu wenigstens 70 Gew.-% aus wenigstens einer Verbindungen der Formel I bestehen, sehr viel einfacher pulverförmige Zusammensetzungen von Astaxanthin-Derivaten herstellen als bei Verwendung des Astaxanthins selber oder anderer, davon verschiedener Astaxanthin-Derivate wie Astaxanthindiacetat, Astaxanthindisuccinat oder Astaxanthindipalmitat.

Die erfindungsgemäßen Zusammensetzungen haben neben einer guten Verdünnbarkeit in den für Nahrungsmittel geeigneten Ölen den Vorteil einer verbesserten Handhabbarkeit und Stabilität im Vergleich zu den aus PCT/EP2008/061384 bekannten flüssigen Suspensionen und zeichnen sich außerdem durch den höheren Gehalt an Astaxanthin-Derivat aus.

In Formel I steht C₁-C₄-Alkyl für einen linearen oder verzweigten, gesättigten Kohlenwasserstoffrest mit 1 bis 4 C-Atomen wie Methyl, Ethyl, n-Propyl, 1-Methylethyl, Butyl, 1-Methylpropyl, 2-Methylpropyl oder 1,1-Dimethylethyl.

Die beiden Reste R in Formel I können gleich oder verschieden sein und sind vorzugsweise identisch.

Vorzugsweise steht A für 1,2-Ethandiyl. R^{x} steht vorzugsweise für Methyl oder Ethyl.

Verbindungen der Formel I, worin A im Rest R für 1,2-Ethandiyl steht, werden im Folgenden auch als Dialkyldisuccinate bezeichnet. Hierunter besonders bevorzugt ist das Dimethylsuccinat.

Die Verbindungen der Formel I können, da die die Gruppe O-C(O)R tragenden Kohlenstoffatome Asymmetriezentren sind, diastereomere und enantiomere Formen bilden, nämlich die 3R,3'S-Form, die 3S,3'R-Form, die 3R,3'R-Form und die 3S,3'S-Form. Sofern die Reste R in Formel I gleich sind, sind die 3R,3'S-Form und die 3S,3R-Form identisch und achiral (meso-Form) und die 3S,3'S-Form und die 3R,3'R-Form bilden ein Enantiomerenpaar. Für die Erfindung können sowohl die reinen Enantiomere und Diastereomere sowie Gemische der vorgenannten Diastereomere, z. B. ein racemisches Gemisch der 3S,3'S-Form und der 3R,3'R-Form als auch ein Gemisch der meso-Form mit der 3S,3'S-Form und/oder der 3R,3'R-Form oder ein Gemisch der meso-Form und dem Racemat der 3S,3'S-Form und der 3R,3'R-Form eingesetzt werden. In einer bevorzugten Ausgestaltung der Erfindung wird die Verbindung der Formel I in der 3S,3'S-Form oder einer Mischung der 3S,3'S-Form mit einer oder weiteren Formen der Verbindung der Formel I eingesetzt, worin die 3S,3'S-Form wenigstens 80 Gew.-%, insbesondere wenigstens 90 Gew.-%, bezogen auf die Gesamtmenge der Verbindung der Formel I ausmacht. In einer weiteren bevorzugten Ausgestaltung der Erfindung wird die Verbindung der Formel I in der 3R,3'R-Form oder einer Mischung der 3R,3'R-Form mit einer oder weiteren Formen der Verbindung der Formel I eingesetzt, worin die 3R,3'R-Form wenigstens 80 Gew.-%, insbesondere wenigstens 90 Gew.-%, bezogen auf die Gesamtmenge der Verbindung der Formel I ausmacht.

In den erfindungsgemäßen Zusammensetzungen weist das Astaxanthin-Derivat üblicherweise eine Reinheit, bezogen auf das in Formel I gezeigten all-trans-Isomer, von wenigstens 70 Gew.-%, häufig wenigstens 80 Gew.-%, vorzugsweise wenigstens 90 Gew.-% und insbesondere wenigstens 95 Gew.-% auf. Neben dem all-trans-Isomeren kann das Astaxanthin-Derivat auch Anteile an Astaxanthin-Verbindungen der Formel I enthalten, in denen eine oder mehrere Doppelbindungen der in Formel I dargestellten all-trans-Isomere cis-Konfiguration aufweisen. Die Gesamtmenge an all-trans-Isomeren der Formel I und cis-Isomeren der Formel I beträgt in der Regel wenigstens 80 Gew.-%, häufig wenigstens 90 Gew.-% und insbesondere wenigstens 96 Gew.-%, bezogen auf das in der Zusammensetzung enthaltene Astaxanthin-Derivat. Neben dem all-trans-Isomeren der Formel I und etwaigem cis-Isomeren kann das Astaxanthin-Derivat auch weitere Carotinoide umfassen, wobei der Anteil dieser Verunreinigungen in der Regel 30 Gew.-%, häufig 20 Gew.-%, insbesondere 10 Gew.- %, besonders bevorzugt 4 Gew.-%, bezogen auf die Gesamtmenge des Astaxanthin-Derivats nicht überschreitet. Bei diesen Verunreinigungen handelt es sich in erster Linie um Halbester des Astaxanthins, d. h. um Verbindungen der im Folgenden gezeigten Formel II 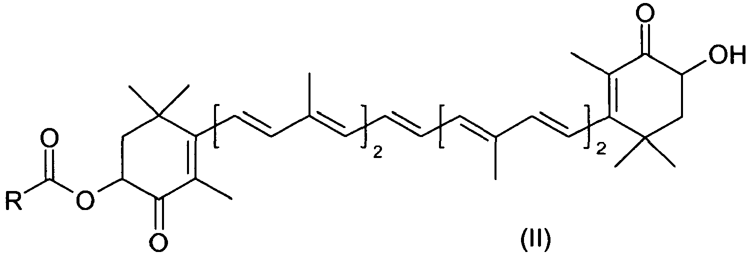 worin R die zuvor genannten Bedeutungen hat bzw. um cis-Isomere davon, sowie um Astaxanthin selber, Adonirubin und/oder Semi-Astacin.

Insbesondere enthält die erfindungsgemäße Zusammensetzung ein Astaxanthin-Derivat, das die an ein für Nahrungsmittel (d. h. für Lebens- und/oder Futtermittel) zugelassenes Astaxanthin gestellten Anforderungen erfüllt, d. h. das weniger als 5 Gew.- % Carotinoide, die von Astaxanthin und seinen Derivaten verschieden sind, enthält und das einen Schwermetallgehalt von höchstens 10 ppm aufweist, das zu wenigstens 70 Gew.-%, häufig zu wenigstens 80 Gew.-%, vorzugsweise zu wenigstens 90 Gew.- %, insbesondere zu wenigstens 96 Gew.-%, bezogen auf die Gesamtmenge an Astaxanthin-Derivat in der Zusammensetzung, aus einer Astaxanthin-Verbindung der Formel I besteht, wobei das in der Zusammensetzung enthaltene Astaxanthin-Derivat zu wenigstens 70 Gew.-%, häufig wenigstens 80 Gew.-%, vorzugsweise wenigstens 90 Gew.-% und insbesondere wenigstens 95 Gew.-% aus dem all-trans-Isomeren besteht.

Die erfindungsgemäßen Formulierungen enthalten vorzugsweise wenigstens 60 Gew.- %, insbesondere wenigstens 70 Gew.-%, besonders bevorzugt wenigstens 80 Gew.-%, speziell wenigstens 90 Gew.-% oder wenigstens 95 Gew.-%, bezogen auf das Gesamtgewicht der Formulierung, wenigstens eines feinteiligen Astaxanthin-Derivats, das zu wenigstens 70 Gew.-%, häufig zu wenigstens 80 Gew.-%, vorzugsweise zu wenigstens 90 Gew.-%, insbesondere zu wenigstens 96 Gew.-%, bezogen auf die Gesamtmenge an Astaxanthin-Derivat in der Zusammensetzung, aus einer Astaxanthin-Verbindung der Formel I besteht.

Neben dem Astaxanthin-Derivat kann die Zusammensetzung auch andere Pulverbestandteile enthalten. Hierzu zählen insbesondere Rieselhilfsmittel und Füllstoffe. Unter einem Rieselhilfsmittel, das auch als Agglomerationshilfsmittel bezeichnet wird, versteht man eine feste, pulverförmige Substanz, die ein Verkleben der Pulverpartikel untereinander oder mit Behälterwänden verringert.

Bei den Rieselhilfsmitteln handelt es sich typischerweise um inerte, für Nahrungsmittel geeignete feste anorganische Materialien in Pulverform, z. B. um Oxide wie Aluminiumoxid, Siliciumdioxid, insbesondere in Form von feinteiliger Kieselsäure wie pyrogene Kieselsäure oder Kieselgel (E 551), oder Titandioxid (E 171), Hydroxide wie Aluminiumhydroxid, Carbonate und Hydrogencarbonate wie Natriumcarbonat (E 500), Natriumhydrogencarbonat, Kaliumcarbonat (E 501), Magnesiumcarbonat (E 504) und Calciumcarbonat (E 170), oder Silikate wie Natriumsilikat (E 550), Magnesiumsilikat (E 553a), Talk (E 553b), Calciumsilikat (E 552), Zinksilikat (E 557), Aluminiumsilikate wie Natriumaluminiumsilikat (554), Kaliumaluminiumsilikat (E 555), Calciumaluminiumsilikat (E 556), Bentonit (E 558), Kaolin (E 559), Natriumchlorid, und/oder um inerte, für Nahrungsmittel geeignete feste organische Materialien in Pulverform, wie beispielsweise kristalline Mono-, Di- und höhere Polysacharide wie Lactose, Zucker (Saccharose), Cellulosepulver, Stearinsäure oder Stearate wie Magnesiumstearat (E 572), Ammoniumstearat (E 571) und Aluminiumstearat (E 573).

Vorzugsweise weisen die Rieselhilfsmittel Teilchengrößen auf, die unterhalb der Teilchengröße der Pulverteilchen liegen. Vorzugweise weisen die Teilchen des Rieselhilfsmittels volumenmittlere Teilchendurchmesser im Bereich von 1/100 bis 1/2 des volumenmittleren Teilchendurchmessers der Pulverteilchen des Astaxanthin-Derivats auf.

Der Anteil der Rieselhilfsmittel wird in der Regel 10 Gew.% nicht überschreiten und beträgt, sofern vorhanden, typischerweise 0,1 bis 10 Gew.-%, vorzugsweise 0,2 bis 5 Gew.-%, insbesondere 0,3 bis 3 Gew.-%, bezogen auf das Gesamtgewicht der Zusammensetzung.

Die erfindungsgemäße Zusammensetzung kann daneben weitere Pulverbestandteile enthalten. Hierzu zählen neben den vorgenannten Rieselhilfsmitteln insbesondere andere organische und anorganische Substanzen, welche für Nahrungsmittel geeignet sind und die vorzugsweise bei 50 °C, insbesondere bei 80 °C ein Feststoff sind. Hierzu zählen insbesondere organische Materialien wie Stärke oder Zellulosepulver. Die Teilchendurchmesser der weiteren Bestandteile liegen in der Regel in den für das Astaxanthin-Derivat genannten Bereichen oder können auch darüber liegen. Vorzugsweise wird die Gesamtmenge an Rieselhilfsmitteln und weiteren Bestandteilen 50 Gew.-%, insbesondere 30 Gew.-%, besonders bevorzugt 20 Gew.-%, speziell 10 Gew.-% oder 5 Gew.-%, bezogen auf das Gesamtgewicht der Formulierung, nicht überschreiten.

Bei den weiteren Bestandteilen kann es sich beispielsweise um
- inerte, für Nahrungsmittel geeignete anorganische Materialien in Pulverform (Füllstoffe), z. B. um Oxide wie Aluminiumoxid, Siliciumdioxid, Titandioxid, Silikate wie Natriumsilikat, Magnesiumsilikat, Talk, Calciumsilikat, Zinksilikat, Aluminiumsilikate wie Natriumaluminiumsilikat, Kaliumaluminiumsilikat, Calciumaluminiumsilikat, Bentonit, Kaolin, Natriumchlorid, sowie um inerte, für Nahrungsmittel geeignete organische Materialien, z. B. Kohlehydrate wie Mono-, Di-, Oligo- und Polysacharide, z. B. Cellulosepulver, Stärke, Lactose und Zucker,
- Antioxidationsmittel (= Antioxidantien) wie beispielsweise α-Tocopherol, α-Tocopherolpalmitat, α-Tocopherolacetat, tert.-Butylhydroxytoluol, tert.-Butylhydroxyanisol, Ascorbinsäure, deren Salze und Ester wie z. B. Natriumascorbat, Calciumascorbat, Ascorbylphosphatester, Fettsäureester der Ascorbinsäure wie Ascorbylstearat und Ascorbylpalmitat sowie Ethoxyquin, sowie um
- Konservierungsmittel wie beispielsweise Benzoesäure und ihre Salze, insbesondere ihre Natrium-, Kalium- und Calciumsalze, 4-Hydroxybenzoesäure (PHB) und ihre Salze, insbesondere ihre Natrium-, Kalium- und Calciumsalze, die Salze der PHB-Alkylester wie das Natriumsalz des PHB-Methylesters, das Natriumsalz des PHB-Ethylesters und das Natriumsalz des PHB-Propylesters, Sorbinsäure und ihre Salze, insbesondere ihre Natrium-, Kalium- und Calciumsalze, Salze der Propionsäure wie insbesondere ihre Natrium-, Kalium- und Calciumsalze, Borsäure und Milchsäure und deren Salze;
   handeln. Der Anteil der weiteren Bestandteile wird 50 Gew.%, bezogen auf das Gesamtgewicht der Zusammensetzung, nicht überschreiten und beträgt, sofern vorhanden, typischerweise 0,1 bis 50 Gew.-%, vorzugsweise 0,1 bis 30 Gew.-%, insbesondere 0,1 bis 20 Gew.-%, 0,1 bis 10 Gew.-% oder 0,1 bis 5 Gew.-%, jeweils bezogen auf das Gesamtgewicht der Zusammensetzung.

In den erfindungsgemäßen Zusammensetzungen weisen wenigstens 90 Gew.-% der Pulverteilchen einen Teilchendurchmesser unterhalb 100 µm, insbesondere maximal 50 µm und speziell maximal 20 µm auf. Die Pulverteilchen weisen erfindungsgemäß eine mittlere Teilchengröße (Teilchendurchmesser, Volumenmittel, D_{4,3}-Wert) im Bereich von 0,5 bis 50 µm, insbesondere im Bereich von 0,5 bis 30 µm und speziell im Bereich von 0,5 bis 10 µm auf.

Die Teilchengrößenverteilung in der Zusammensetzung kann monomodal oder polymodal sein, d. h. die Verteilungskurve kann ein Maximum (monomodale Verteilung) oder mehrere Maxima aufweisen. Bevorzugt ist der Anteil der Partikel des Astaxanthin-Derivats mit Teilchengrößen oberhalb 50 µm, insbesondere oberhalb 20 µm gering und macht vorzugsweise nicht mehr als 20 Gew.-%, insbesondere nicht mehr als 10 Gew.- %, bezogen auf die Gesamtmenge des Astaxanthin-Derivats in der Zusammensetzung aus. Die Teilchengröße und Teilchengrößenverteilung eines gegebenenfalls in der Zusammensetzung enthaltenen Rieselhilfsmittels oder eines sonstigen festen Bestandteils ist hingegen von untergeordneter Bedeutung. Insbesondere kann die mittlere Teilchengröße der Partikel des Rieselhilfsmittels oder des sonstigen festen Bestandteils von der mittleren Teilchengröße der Partikel des Astaxanthin-Derivats abweichen.

Unter dem der volumenmittleren Teilchengröße versteht man den volumenmittleren Teilchendurchmesser (D_{4,3}-Wert), wie man ihn mittels Fraunhofer-Beugung bestimmen kann.

Die erfindungsgemäßen Zusammensetzungen können in einfacher Weise durch trockenes Vermahlen des in der Zusammensetzung enthaltenen Astaxanthin-Derivats auf die erfindungsgemäße Partikelgröße hergestellt werden. Dementsprechend betrifft die vorliegende Erfindung auch ein Verfahren zur Herstellung einer pulverförmigen Zusammensetzung wie hier beschrieben, umfassend trockenes Vermahlen eines Astaxanthin-Derivats, das zu wenigstens 70 Gew.-% aus einer Astaxanthin-Verbindung der Formel I besteht.

Im Unterschied zur Nassmahlung, bei der eine Suspension des Mahlguts in einem geeigneten flüssigen Verdünnungsmittel vermahlen wird, versteht man unter einem trockenen Vermahlen ein Vermahlen des Mahlgutes, hier des festen Astaxanthin-Derivats und gegebenenfalls weiterer fester Bestandteile der Zusammensetzung, als Feststoff, d. h. in Abwesenheit eines flüssigen Verdünnungsmittels. Mit anderen Worten, beim trockenen Vermahlen, wird das Mahlgut als Feststoff, z. B. mittels Schwerkraft oder vorzugsweise mittels eines Gasstroms, in die Mahlzone eingebracht, auf die gewünschte Teilchengröße zerkleinert, und anschließend aus der Mahlzone ausgetragen, z. B. mittels eines Gasstroms. Gegebenenfalls kann sich hieran eine Maßnahme zur Diskriminierung des gemahlenen Mahlgutes hinsichtlich der Partikelgröße anschließen, wobei grobteiliges Mahlgut, welches noch nicht die gewünschte Feinheit aufweist, abgetrennt und in den Mahlprozess rückgeführt werden kann.

Bevorzugt sind Mahlvorrichtungen, bei denen der Zerkleinerungsvorgang im Wesentlichen auf einem gegenseitigen Austausch von kinetischer Energie der Mahlgutpartikel untereinander beruht. Hierbei wird das Mahlgut mit Hilfe eines oder mehrerer Gasströme in einer Mahlkammer so beschleunigt, dass die Partikel aufeinanderprallen und so einer Zerkleinerung erreicht wird. Beispiele für derartige Mahlvorrichtungen sind Strahlmühlen, wie insbesondere Luftstrahlmühlen, Gegenstrahlmühlen und Spiralstrahlmühlen, die jeweils mit Mitteln zur Klassierung der Partikel des Mahlgutes (Sichter) und gegebenenfalls Rückführung des Mahlgutes, wie beispielsweise Abweiserad-Sichter, kombiniert sein können. Die Mühlen können auch mit nachgeordneten Vorrichtungen zur Klassierung und gegebenenfalls Rückführung des Mahlgutes kombiniert sein, wie beispielsweise Zyklone. Bei den Gasströmen kann es sich um Luft, z. B. Pressluft, oder um Inertgas, z. B. Stickstoff, oder um eine Mischung aus Luft und Inertgas handeln.

Es hat sich als besonders vorteilhaft erwiesen, das feste Astaxanthin-Derivat mittels einer so genannten Spiralstrahlmühle auf die gewünschte Partikelgröße zu zerkleinern. Unter Spiralstrahlmühlen versteht man Mahlvorrichtungen, die eine im Wesentlichen zylindrische Mahlkammer mit kreisförmigem oder vorzugsweise elliptischem Grundriss aufweisen, deren Umfang mehrere, gleichmäßig über den Umfang verteilte Strahldüsen aufweist, durch die mit hohem Druck von in der Regel 1 bis 16 bar (Überdruck) ein Gasstrom in die Mahlkammer geleitet wird. Die Düsen sind unter einem spitzen Winkel zur Tangente angestellt (in der Regel 25° bis 80°) und erzwingen in der Mahlkammer eine Spiralströmung. Das Mahlgut wird über eine geeignete Zuführung in die Zerkleinerungszone der Mahlkammer eingespeist und vom Gasstrom beschleunigt. Die Zerkleinerung beruht hierbei im Wesentlichen auf dem Austausch kinetischer Energie der Mahlgutpartikel. Im Anschluss an die durch Teilchenstoß erzielte Zerkleinerung schließt sich zur Mahlkammermitte eine Sichtzone der Spiralströmung an. Ein Teil der nicht genügend zerkleinerten Partikel wird durch Zentrifugalkräfte zurückgehalten und gelangt wiederum in den Zerkleinerungsbereich. Vorzugsweise weist die Spiralstrahlmühle einen integrierten Sichter, beispielsweise ein steuerbares Sichtrad, auf, um die Kornobergrenze möglichst einheitlich zu halten.

Derartige Vorrichtungen sind aus dem Stand der Technik bekannt, z. B. aus der US 3,425, 638, der GB 1098546, der WO 96/01694 und kommerziell verfügbar, z. B. von den Firmen Sweco Europe SA (Nivelles, Belgien), Hosokawa Alpine AG (Augsburg, Deutschland), Micro-Macinazione SA (Molinazzo di Monteggio, Schweiz), PMT Jetmilll GmbH (Kammern, Österreich), Netzsch CONDUX Mahltechnik GmbH (Hanau, Deutschland).

Ebenfalls geeignet sind Gegenstrahlmühlen, einschließlich Fließbettgegenstrahlmühlen. Hierunter versteht man Mahlvorrichtungen, bei denen über zwei oder mehrere gegeneinander gerichtete Düsen ein Gasstrom in die Mahlkammer geleitet wird, welcher das Mahlgut fluidisiert. Hierdurch werden die Teilchen des Mahlgutes beschleunigt und prallen im zentral gelegenen Überschneidungsbereich der Strahlen aufeinander, wodurch sie zerkleinert werden. Gleichzeitig bilden die Luftstrahlen im Überscheidungsbereich eine nach oben gerichtete Strömung aus, welche das zerkleinerte Mahlgut nach oben in einer Sichterzone transportiert. Ein Teil der nicht genügend zerkleinerten Partikel wird durch die Schwerkraft zurückgehalten und verbleibt in dem Zerkleinerungsbereich. Das Mahlgut gelangt in der Regel von oben über eine geeignete Zuführung in den Zerkleinerungsbereich der Mahlkammer. Die Zerkleinerung beruht auch hier im Wesentlichen auf dem Austausch kinetischer Energie der Mahlgutpartikel. Vorzugsweise weist die Gegenstrahlmühle einen integrierten Sichter, beispielsweise ein steuerbares Sichtrad, auf, um die Kornobergrenze möglichst einheitlich zu halten.

Derartige Vorrichtungen sind aus dem Stand der Technik bekannt und kommerziell erhältlich, z. B. von den Firmen Attritor Ltd. (Edgwick Park, Coventry, UK), Hosokawa Alpine AG (Augsburg, Deutschland), Netzsch CONDUX Mahltechnik GmbH (Hanau, Deutschland), Noll GmbH (Bobingen, Deutschland).

Das Vermahlen des Astaxanthin-Derivats kann in Gegenwart weiterer Bestandteile der Zusammensetzung erfolgen. Hierbei handelt es sich typischerweise um die oben genannten weiteren Bestandteile wie inerte, für Nahrungsmittel geeignete Bestandteile (Füllstoffe) sowie um Konservierungsmittel und Antioxidantien.

Der Anteil der weiteren Bestandteile wird 50 Gew.%, bezogen auf das Gesamtgewicht der Zusammensetzung, nicht überschreiten und beträgt, sofern vorhanden, typischerweise 0,1 bis 50 Gew.-%, vorzugsweise 0,1 bis 30 Gew.-%, insbesondere 0,1 bis 20 Gew.-% oder 0,1 bis 10 Gew.-%, jeweils bezogen auf das Gesamtgewicht der Zusammensetzung.

Die erfindungsgemäßen festen Zusammensetzungen lassen sich einfach in flüssige, für Nahrungsmittel geeignete Öle einarbeiten und eigenen sich daher in besonderer Weise zur Herstellung von Futtermittelzubereitungen, insbesondere festen Futtermittelzubereitungen, speziell Futtermittelzubereitungen in Form von Pellets.

Dementsprechend betrifft die vorliegende Erfindung auch ein Verfahren zur Herstellung von Futtermittelzubereitungen, insbesondere von festen Futtermittelzubereitungen, speziell von Pellets, umfassend die folgenden Schritte:
a) Bereitstellung einer erfindungsgemäßen, pulverförmigen Zusammensetzungen wie hier beschrieben;
b) Lösen der Zusammensetzung in einem für Nahrungsmittel geeigneten flüssigen Öl unter Erhalt einer verdünnten flüssigen Lösung des Astaxanthin-Derivats in dem flüssigen Öl; und
c) Vermischen der in Schritt b) erhaltenen flüssigen Lösung mit den Bestandteilen der Futtermittelzubereitung oder Imprägnieren einer festen Futtermittelzubereitung mit der in Schritt b) erhaltenen flüssigen Lösung.

Das in Schritt b) eingesetzte Öl kann synthetischer, mineralischer, pflanzlicher oder tierischer Herkunft sein. Beispiele sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/- Caprinsäure-Tricyceride wie die Miglyol-Typen, weiterhin Paraffinöl, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl.

Bevorzugt sind pflanzliche Öle und Öle tierischen Ursprungs, die bei 40 °C flüssig sind, insbesondere Pflanzenöle wie Sojabohnenöl, Sonnenblumenöl, Distelöl, Maiskeimöl, Olivenöl, Leinsamenöl, Rapsöl, Reisöl, Kokosöl, Erdnussöl, Palmöl, Palmkernöl, PUFA-Öle, MCT-Öle, weiterhin Fischöle, sowie Mischungen dieser Öle.

In einer bevorzugten Ausführungsform der Erfindung umfasst das in Schritt b) eingesetzte Öl zu wenigstens 50 Gew.-% und insbesondere zu wenigstens 80 Gew.-%, bezogen auf die Gesamtmenge des in Schritt b) eingesetzten essbaren Öls, wenigstens ein unter Maisöl, Sonnenblumenöl, Sojabohnenöl und Fischöl ausgewähltes essbares Öl.

Bei den in Schritt b) eingesetzten Ölen kann es sich um Raffinatöle oder Rohöle handeln, die noch ursprungsspezifische Verunreinigungen wie Proteine, Phosphat, (Erd-)Alkalimetallsalze und dergleichen in üblichen Mengen enthalten. Die in Schritt b) eingesetzten Öle können auch geringe Mengen an emulgiertem und/oder gelöstem Wasser, vorzugsweise jedoch nicht mehr als 10 Gew.-%, insbesondere nicht mehr als 1 Gew.-%, bezogen auf die Gesamtmenge des in Schritt b) eingesetzten Öls, enthalten.

Das Vermischen des Öls mit der pulverförmigen Zusammensetzung erfolgt typischerweise bei Temperaturen unterhalb 100 °C, vorzugsweise bei Temperaturen von nicht mehr als 95 °C und speziell nicht mehr als 90 °C und vorzugsweise bei Temperaturen im Bereich von 20 bis < 100°C, insbesondere im Bereich von 25 bis 95 °C und speziell im Bereich von 30 bis 90 °C.

Vorzugsweise wird man das Öl, in welches man die pulverförmige Zusammensetzung des Astaxanthin-Derivats einarbeitet, erwärmen. In der Regel liegt die Temperatur des erwärmten Öls vorzugsweise unterhalb 100 °C und beträgt insbesondere nicht mehr als 95 °C und speziell nicht mehr als 90 °C und liegt vorzugsweise im Bereich von 25 bis < 100 °C, insbesondere im Bereich von 30 bis 95 °C und speziell im Bereich von 35 bis 90 °C.

Typischerweise wird man die relativen Mengen an Öl und erfindungsgemäßer Zusammensetzung so wählen, dass der Gehalt an Astaxanthin-Derivat in der resultierenden Lösung im Bereich von 10 ppm (= 0,001 Gew.-%) bis etwa 1 Gew.-%, häufig im Bereich von 20 ppm bis 0,5 Gew.-%, insbesondere im Bereich von 50 ppm bis 0,2 Gew.- %, bezogen auf die Gesamtmenge an Öl plus Astaxanthin-Derivat liegt.

Beispielsweise kann man so vorgehen, dass man ein für Nahrungsmittel geeignetes Öl, das vorzugsweise einen Schmelzpunkt von nicht mehr als 40 °C aufweist, in einem geeigneten Gefäß vorlegt, gegebenenfalls auf die gewünschte Temperatur vorwärmt und hierzu die erfindungsgemäße Zusammensetzung unter Durchmischen zugibt, wobei die Zugabe in einer Portion, in mehreren Portionen oder kontinuierlich erfolgen kann. Die zugegebene erfindungsgemäße Zusammensetzung kann auf die gewünschte Temperatur vorgewärmt werden, was jedoch grundsätzlich nicht erforderlich ist.

Die zum Erreichen des Lösens erforderliche Zeit hängt naturgemäß von der Temperatur, dem gewählten Öl und den apparativen Gegebenheiten ab und liegt typischerweise im Bereich von 1 min bis 120 min. Der Fachmann kann die erforderliche Zeit durch Routineexperimente ermitteln.

Im Anschluss an das Lösen der erfindungsgemäßen Zusammensetzung kann man die erhaltene ölige Lösung, sofern erforderlich, abkühlen, z. B. mittels geeigneter Wärmetauscher oder durch Verdünnen mit einem kalten essbaren Öl, das zu dem zum Lösen in Schritt b) verwendeten Öl gleich oder verschieden sein kann.

Auf diese Weise erhält man stabile Lösungen von Astaxanthin-Derivaten in für Nahrungsmittel geeigneten Ölen. Diese Lösungen zeichnen sich dadurch aus, dass das in ihnen enthaltene Astaxanthin-Derivat einen hohen Anteil an all-trans-Isomeren aufweist, der in der Regel oberhalb 80 %, insbesondere oberhalb 90 % liegt.

Die so erhaltenen Lösungen enthalten neben dem Astaxanthin-Derivat die in den Zusammensetzungen enthaltenen Hilfsstoffe, wie Mahl- oder Rieselhilfsmittel, die sich nicht notwendigerweise lösen müssen, sowie ggf. weitere Zusätze, wie Oxidationsstabilisatoren und lipophile Dispergiermittel.

Es versteht sich von selber, dass die in Schritt b) erhaltenen Lösungen geringe Mengen an Wasser aus dem zu ihrer Herstellung eingesetzten, für Nahrungsmittel Öl enthalten können. Häufig jedoch beträgt der Wasseranteil nicht mehr als 10 Gew.-%, z. B. 0,1 bis 10 Gew.-%, häufig 0,5 bis 8 Gew.-%, und in einer speziellen Ausführungsform der Erfindung nicht mehr als 0,5 Gew.-%, bezogen auf das in der Lösung enthaltene Öl.

Die nach dem erfindungsgemäßen Verfahren erhältlichen Lösungen des Astaxanthin-Derivats sind lagerstabil und können vor ihrer weiteren Verwendung über längere Zeiträume aufbewahrt werden, ohne dass es in nennenswertem Umfang zu einem Aktivitätsverlust, z. B. durch Isomerisierung und/oder oxidativen Abbau, kommt. Insbesondere zeichnen sie sich durch einen hohen Anteil an all-trans-Isomeren des eingesetzten Astaxanthin-Derivats aus und durch einen vergleichsweise geringen Anteil an Abbauprodukten des Astaxanthin-Derivats.

Das erfindungsgemäße Verfahren wird häufig direkt in die Prozesse zur Weiterverarbeitung der Lösungen des Astaxanthin-Derivats integriert werden.

Die nach dem erfindungsgemäßen Verfahren erhältlichen Lösungen des Astaxanthin-Derivats eignen sich in vorteilhafter Weise als Zusatz zu Tierfuttermitteln, Nahrungsmitteln für die Humanernährung, Nahrungsergänzungsmitteln, pharmazeutischen Mitteln oder kosmetischen Mitteln. Bevorzugt lassen sich die Lösungen als Futtermittelzusatz in der Tieremährung einsetzen, beispielsweise bei der Futtermittelherstellung durch Einmischen in einen Futtermittelteig vor oder während der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als so genannte "post-pelleting liquid application". Vorzugsweise belädt man die Futtermittelpellets mit den Formulierungen unter vermindertem Druck.

Dementsprechend betrifft die vorliegende Erfindung auch die Herstellung von Futtermitteln, wie Tierfuttermittel, Nahrungsmittel für die Humanernährung, Nahrungsergänzungsmittel, sowie die Herstellung pharmazeutischer Mittel oder kosmetischer Mittel unter Verwendung der erfindungsgemäß hergestellten Lösungen des Astaxanthin-Derivats. Diese Mittel enthalten neben den für diese Mittel üblichen Bestandteilen das bei 30 °C flüssige Öl, das feste Verdünnungsmittel und das Astaxanthin-Derivat.

Bevorzugte Ausführungsformen betreffen Tierfuttermittel, insbesondere Fischfuttermittel, die das bei 30 °C flüssige Öl, das feste Verdünnungsmittel und das Astaxanthin-Derivat umfassen. Derartige Mittel enthalten das in der Lösung enthaltene Astaxanthin-Derivat typischerweise in einer Menge von 10 bis 100 ppm, bezogen auf das Gesamtgewicht des Mittels, wobei das Astaxanthin-Derivat in der Regel zu mehr als 70 %, insbesondere zu wenigstens 80 % und speziell zu wenigstens 90 % eine all-trans-Konfiguration aufweist.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs, weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen, und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z. B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Eine spezielle Ausführungsform dieser Mittel betrifft Futtermittelpellets, speziell Futtermittelpellets für Fischfutter, die mit der erfindungsgemäß erhältlichen Lösung des Astaxanthin-Derivats beladen sind. Derartige Pellets enthalten das in der Lösung enthaltene Astaxanthin-Derivat typischerweise in einer Menge von 10 bis 100 ppm, bezogen auf das Gesamtgewicht des Futtermittels. Die Herstellung solcher Pellets erfolgt in der Regel durch Besprühen konventioneller Pellets mit einer erfindungsgemäß erhältlichen Lösung des Astaxanthin-Derivats, vorzugsweise unter vermindertem Druck, wobei das Besprühen kontinuierlich oder vorzugsweise diskontinuierlich erfolgen kann. Beispielsweise kann man die konventionellen Pellets in einem geeigneten Gefäß vorlegen, das Gefäß evakuieren und dann Öl unter Durchmischen der Pellets aufsprühen und anschließend belüften. Auf diese Weise erreicht man ein gleichmäßiges Eindringen der erfindungsgemäßen öligen Zusammensetzung in die Pellets. Gegebenfalls kann man erneut Vakuum anlegen und erneut die Lösung des Astaxanthin-Derivats oder ein für Nahrungsmittel geeignetes, flüssiges Öl in der zuvor beschriebenen Weise aufsprühen. Auf diese Weise erhält man Pellets, die im Kern das Öl und das Astaxanthin-Derivat enthalten.

Die folgenden Beispiele dienen der weiteren Erläuterung der Erfindung.

In den folgenden Versuchen wurde das in Beispiel 5 der WO 03/066583 beschriebene (all-E)-3,3'-rac-Astaxanthindimethyldisuccinat (Ax-DMDS) in kristalliner Form mit einer Reinheit von > 96 % eingesetzt (Verbindung der Formel I, worin R für C(=O)CH₂CH₂C(=O)OCH₃ steht).

Zur Bestimmung des relativen Gehalts an gelöstem Astaxanthin-Derivat in den erfindungsgemäßen Lösungen, bezogen auf die eingesetzte Menge an Astaxanthin-Derivat, filtrierte man die Proben über einen Filter mit einer Porenweite von 0,2 µm und bestimmte anschließend mittels UV/VIS-Spektroskopie (200-800 nm) die Extinktion der Lösung bei 478 nm. Hierbei wurde für Ax-DMDS eine Extinktion E1/1 von 1250 zugrunde gelegt.

### Herstellung von Pulvern durch Trockenmahlen von kristallinem Ax-DMDS:

Die folgenden Trockenmahlversuche wurden mit einer Labor-Spiralstrahlmühle mit zylindrischer Mahlkammer mit einem Durchmesser von 40 mm und einer Höhe von 10 mm durchgeführt, wobei die Mahlkammer 4 zylindrische Mahldüsen im Winkel von 53° und einem Durchmesser von 0,6 mm aufwies. Die Spiralstrahlmühle wies einen Injektor mit 2 Treibdüsen (0,7 mm), sowie einen Auslass für das Mahlgut mit angeschlossenem Filter auf.

Die Bestimmung der Partikelgröße erfolgte durch Laserlichtstreuung im Gastrom mit einem Mastersizer S der Fa. Malvern. Hierzu wurde das Pulver über ein Injektorsystem "Rodos" der Fa. Sympatek in den Messbereich des Malvern Mastersizer S eingeblasen. Die Zuführung in das Injektorsystem erfolgte über eine Dosierrinne in den Trichter des Injektorsystems. Die Injektordüse wurde mit einem Gasdruck von 1 bar betrieben. Das Injektorsystem verfügte über eine Venturidüse zur Bestimmung des Drucks zur Einstellung reproduzierbarer Messbedingungen (Volumenstrom, Bestimmung der Gasgeschwindigkeit, hier 120 m/s, 1170 Pa). Die Messkonzentration wird über die Abschattung im Messvolumen angegeben und lag im Bereich von 1 bis 4 %.

### Beispiel 1:

11 g Ax-DMDS wurden in die Ansaugzone des Injektors der oben beschriebenen Spiralstrahlmühle gegeben. Der Mahldruck betrug 6 bar, der Injektordruck 7 bar. Der Gasdurchsatz lag bei 10 m³/h.

Das erhaltene Pulver wies folgende Partikelgrößenverteilung auf:
D₁₀: 1,6 µm,
D₅₀: 4,9 µm,
D₉₀; 15 µm.

### Beispiel 2:

10 g Ax-DMDS wurden in die Ansaugzone des Injektors der oben beschriebenen Spiralstrahlmühle gegeben. Der Mahldruck betrug 4 bar, der Injektordruck 5 bar. Der Gasdurchsatz lag bei 7 m³/h.

Das erhaltene Pulver wies folgende Partikelgrößenverteilung auf:
D₁₀: 1,9 µm,
D₅₀: 7,9 µm,
D₉₀; 37 µm.

### Herstellung von Lösungen des Ax-DMDS

In den folgenden Löseversuchen wurden die folgenden Öle eingesetzt:
MCT-Öl: Delios® MCT-Öl der Fa. Cognis GmbH; Wassergehalt < 0,1 %, Gehalt an C8/C10-Fettsäuretriglyceriden > 95 %.

### Beispiel 3:

0,021 g des Trockenpulvers aus Beispiel 1 wurden bei 60 °C unter Rühren mit einem UltraTurrax in 100 g MCT-Öl suspendiert. Der Anteil der gelösten Bestandteile wurde mittels UVNIS-Spektroskopie ermittelt. Anschließend wurde die Probe 10 min bei 60 °C gelagert, erneut 1 min mit einer Zahnkranzdispergiermaschine (UltraTurrax) suspendiert und der Anteil der gelösten Bestandteile wurde mittels UVNIS-Spektroskopie ermittelt.

In einem parallelen Versuch wurden 0,021 g des Trockenpulvers aus Beispiel 1 bei 60 °C unter Rühren mit einem UltraTurrax in 100 g MCT-Öl suspendiert. Die Probe wurde 180 min bei 60 °C gelagert, erneut 1 min mit UltraTurrax suspendiert und der Anteil der gelösten Bestandteile wurde mittels UVNIS-Spektroskopie ermittelt.

### Vergleichsbeispiel

Zu Vergleichszwecken wurde kristallines Ax-DMDS, das Partikeldurchmesser im Bereich von 0,1 bis 0,5 mm aufwies, in der in Beispiel 3 beschriebenen Weise bei 60 °C suspendiert und anschließend 10 min bzw. 180 min gelagert.

**Tabelle 1**

| Versuchsparameter | Zeit | Ax-DMDS | Ax-DMDS | Ax-DMDS⁴⁾ | Ax-DMDS⁴⁾ |
|---|---|---|---|---|---|
| | [min]¹⁾ | [ppm]²⁾ | [%]³⁾ | [PPm]²⁾ | [%]³⁾ |
| MCT Öl | 0 | 210 | 100 | 129 | 62,6 |
| 60 °C | 10 | 210 | 100 | 202 | 202 |
| 210 ppm | 180 | 210 | 100 | 210 | 210 |

| | | | | | |
|---|---|---|---|---|---|
| 1) Zeitpunkt der Probennahme, nach Suspension mit Ultraturrax 2) Konzentration an gelöstem Ax-DMDS 3) Anteil an gelöstem Ax-DMDS, bezogen auf das in der Suspension enthaltene Ax-DMDS 4) Kristallines Ax-DMDS, ungemahlen | | | | | |

## Patentansprüche

1. Pulverförmige Zusammensetzung, enthaltend wenigstens ein Astaxanthin-Derivat in einer Menge von wenigstens 50 Gew.-%, bezogen auf das Gesamtgewicht der Zusammensetzung, worin wenigstens 90 Gew.-% der Pulverteilchen einen Teilchendurchmesser unterhalb 100 µm aufweisen und worin die Pulverteilchen eine mittlere Teilchengröße (Volumenmittel) im Bereich von 0,5 bis 50 µm aufweisen und
wobei das Astaxanthin-Derivat zu wenigstens 70 Gew.-% aus wenigstens einer Verbindung der Formel I 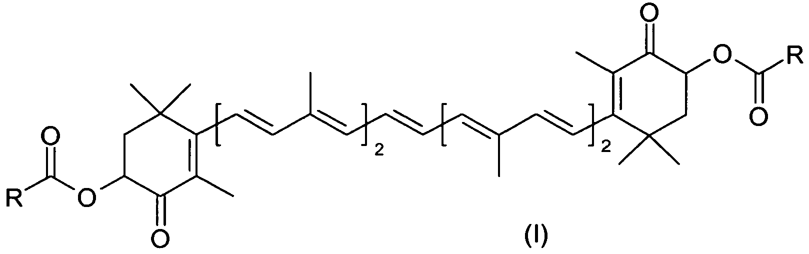 besteht, worin R für einen Rest der Formel 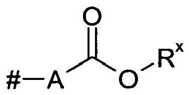 steht, worin # die Anknüpfung an die Carbonylgruppe bedeutet, A für -CH₂CH₂- oder für -CH=CH- steht und R^{x} für C₁-C₄ Alkyl steht.

2. Zusammensetzung nach Anspruch 1, wobei in Formel I der Rest R^{x} Methyl oder Ethyl bedeutet und A für -CH₂CH₂- steht.

3. Zusammensetzung nach Anspruch 1 oder 2, bestehend im Wesentlichen aus dem Astaxanthin-Derivat, das zu wenigstens 70 Gew.-% aus einer Astaxanthin-Verbindung der Formel I besteht, und gegebenenfalls einem oder mehreren festen Rieselhilfsmitteln in einer Menge bis 10 Gew.-%, bezogen auf das Gesamtgewicht der Zusammensetzung.

4. Zusammensetzung nach Anspruch 3, wobei die festen Rieselhilfsmittel ausgewählt sind unter Kieselsäure, Aluminiumoxid, Silikate, Aluminiumsilikate, Titandioxid, Natriumcarbonat, Natriumhydrogencarbonat, Kaliumcarbonat, Magnesiumcarbonat, Calciumcarbonat, Natriumchlorid, Monosacchariden, Disacchariden, Cellulosepulver und Stearaten.

5. Zusammensetzung nach einem der vorhergehenden Ansprüche, erhältlich durch trockenes Vermahlen eines Astaxanthin-Derivats, das zu wenigstens 70 Gew.-% aus einer Astaxanthin-Verbindung der Formel I besteht.

6. Zusammensetzung nach einem der vorhergehenden Ansprüche, worin das Astaxanthin-Derivat zu wenigstens 80 % in der all-trans-Form vorliegt.

7. Verfahren zur Herstellung einer Zusammensetzung gemäß einem der vorhergehenden Ansprüche, umfassend trockenes Vermahlen eines Astaxanthin-Derivats, das zu wenigstens 70 Gew.-% aus einer Astaxanthin-Verbindung der Formel I besteht.

8. Verwendung einer Zusammensetzung nach einem der Ansprüche 1 bis 6 zur Herstellung von Futtermittelzubereitungen, insbesondere festen Futtermittelzubereitungen, speziell Futtermittelzubereitungen in Form von Pellets.

9. Verwendung einer Zusammensetzung nach einem der Ansprüche 1 bis 6 zur Herstellung einer Lösung des Astaxanthin-Derivats in einem für Nahrungsmittel geeigneten Öl.

10. Verfahren zur Herstellung von Futtermittelzubereitungen, insbesondere von festen Futtermittelzubereitungen, speziell von Pellets, umfassend:
a) Bereitstellung einer pulverförmigen Zusammensetzung gemäß einem der Ansprüche 1 bis 6;
b) Lösen der Zusammensetzung in einem für Nahrungsmittel geeigneten, flüssigen Öl unter Erhalt einer verdünnten flüssigen Lösung des Astaxanthin-Derivats in dem flüssigen Öl; und
c) Vermischen der in Schritt b) erhaltenen flüssigen Lösung mit den Bestandteilen der Futtermittelzubereitung oder Imprägnieren einer festen Futtermittelzubereitung mit der in Schritt b) erhaltenen flüssigen Lösung.

## Claims

1. A pulverulent composition comprising at least one astaxanthin derivative in an amount of at least 50% by weight, based on the total weight of the composition, in which at least 90% by weight of the powder particles have a particle diameter below 100 µm and in which the powder particles have a median particle size (volume average) in the range from 0.5 to 50 µm, and
wherein the astaxanthin derivative comprises at least 70% by weight of at least one compound of the formula I 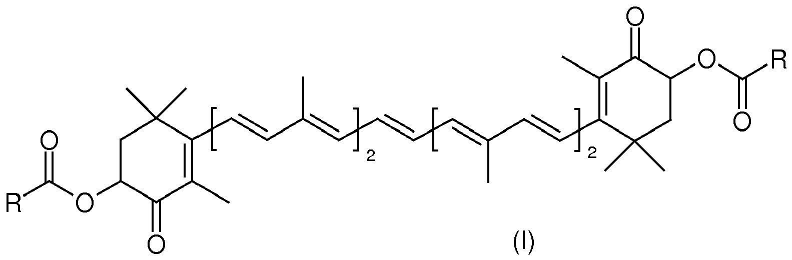 where R is a moiety of the formula 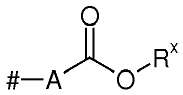 where # means the link to the carbonyl group, A is -CH₂CH₂- or -CH=CH- and R^{x} is C₁-C₄ alkyl.

2. The composition according to claim 1, wherein in formula I the moiety R^{x} is methyl or ethyl and A is -CH₂CH₂- .

3. The composition according to claim 1 or 2, comprising essentially the astaxanthin derivative which comprises at least 70% by weight of an astaxanthin compound of the formula I, and optionally one or more solid anticaking agents in an amount of up to 10% by weight, based on the total weight of the composition.

4. The composition according to claim 3, wherein the solid anticaking agents are selected from silicic acid, aluminum oxide, silicates, aluminum silicates, titanium dioxide, sodium carbonate, sodium hydrogencarbonate, potassium carbonate, magnesium carbonate, calcium carbonate, sodium chloride, monosaccharides, disaccharides, cellulose powders and stearates.

5. The composition according to any one of the preceding claims, obtainable by dry milling an astaxanthin derivative which comprises at least 70% by weight of an astaxanthin compound of the formula I.

6. The composition according to any one of the preceding claims, wherein at least 80% of the astaxanthin derivative is present in the all-trans form.

7. A method for producing a composition according to any one of the preceding claims, which comprises dry milling an astaxanthin derivative which comprises at least 70% by weight of an astaxanthin compound of the formula I.

8. The use of a composition according to any one of claims 1 to 6 for producing feed preparations, in particular solid feed preparations, especially feed preparations in the form of pellets.

9. The use of a composition according to any one of claims 1 to 6 for producing a solution of the astaxanthin derivative in an oil which is suitable for foods.

10. A method for producing feed preparations, in particular solid feed preparations, especially pellets, which comprises:
a) providing a pulverulent composition according to any one of claims 1 to 6;
b) dissolving the composition in a liquid oil which is suitable for foods obtaining a dilute liquid solution of the astaxanthin derivative in the liquid oil; and
c) mixing the liquid solution obtained in step b) with the components of the feed preparation or impregnating a solid feed preparation with the liquid solution obtained in step b).

## Revendications

1. Composition pulvérulente, contenant au moins un dérivé d'astaxanthine en une quantité d'au moins 50 % en poids, par rapport au poids total de la composition, dans laquelle au moins 90 % en poids des particules de poudre présentent un diamètre de particule inférieur à 100 µm et dans laquelle les particules de poudre présentent une taille moyenne de particule (moyenne en volume) dans la plage de 0,5 à 50 µm et
le dérivé d'astaxanthine consistant à raison d'au moins 70 % en poids en au moins un composé de formule I 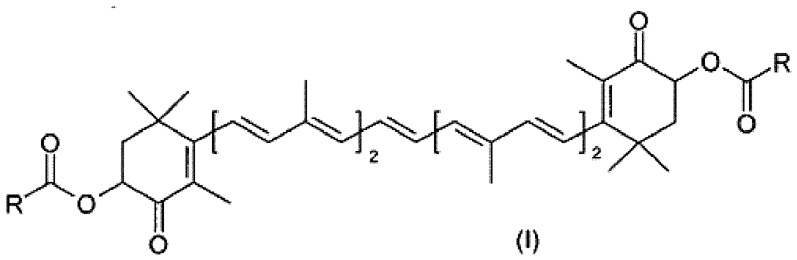 dans laquelle R représente un radical de formule 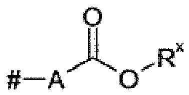 dans laquelle # représente la liaison au groupe carbonyle, A représente -CH₂CH₂- ou -CH=CH- et R^{x} représente un groupe alkyle en C₁-C₄.

2. Composition selon la revendication 1, dans laquelle le radical R^{x} dans la formule I représente le groupe méthyle ou éthyle et A représente -CH₂CH₂-.

3. Composition selon la revendication 1 ou 2, consistant essentiellement en le dérivé d'astaxanthine qui consiste à raison d'au moins 70 % en poids en un composé astaxanthine de formule I, et éventuellement en un ou plusieurs antiagglomérants solides en une quantité allant jusqu'à 10 % en poids, par rapport au poids total de la composition.

4. Composition selon la revendication 3, dans laquelle les antiagglomérants solides sont choisis parmi la silice, l'oxyde d'aluminium, des silicates, des silicates d'aluminium, le dioxyde de titane, le carbonate de sodium, l'hydrogénocarbonate de sodium, le carbonate de potassium, le carbonate de magnésium, le carbonate de calcium, le chlorure de sodium, des monosaccharides, des disaccharides, la poudre de cellulose et des stéarates.

5. Composition selon l'une quelconque des revendications précédentes, pouvant être obtenue par broyage à sec d'un dérivé d'astaxanthine qui consiste à raison d'au moins 70 % en poids en un composé astaxanthine de formule I.

6. Composition selon l'une quelconque des revendications précédentes, dans laquelle le dérivé d'astaxanthine se trouve à raison d'au moins 80 % sous la forme *tout-trans.*

7. Procédé pour la préparation d'une composition selon l'une quelconque des revendications précédentes, comprenant le broyage à sec d'un dérivé d'astaxanthine qui consiste à raison d'au moins 70 % en poids en un composé astaxanthine de formule I.

8. Utilisation d'une composition selon l'une quelconque des revendications 1 à 6, pour la fabrication de préparations d'aliments pour animaux, en particulier de préparations solides d'aliments pour animaux, spécialement de préparations d'aliments sous forme de boulettes pour animaux.

9. Utilisation d'une composition selon l'une quelconque des revendications 1 à 6, pour la préparation d'une solution du dérivé d'astaxanthine dans une huile appropriée pour des produits alimentaires.

10. Procédé pour la fabrication de préparations d'aliments pour animaux, en particulier de préparations solides d'aliments pour animaux, spécialement de boulettes, comprenant :
a) disposition d'une composition pulvérulente selon l'une quelconque des revendications 1 à 6 ;
b) dissolution de la composition dans une huile liquide appropriée pour des produits alimentaires, avec obtention d'une solution liquide diluée du dérivé d'astaxanthine dans l'huile liquide ; et
c) mélange de la solution liquide obtenue dans l'étape b) avec les composants de la préparation d'aliment pour animaux ou imprégnation d'une préparation solide d'aliment pour animaux avec la solution liquide obtenue dans l'étape b).
